# EUROPEAN PATENT SPECIFICATION

(11) **EP 3 722 285 B1**
(45) Date of publication and mention of the grant of the patent: **30.03.2022**
(21) Application number: 20168811.6
(22) Date of filing: 08.04.2020
(51) Int. Cl.: C07D 277/40

(54) **PROCESS FOR PREPARING MIRABEGRON ENACARBIL**
VERFAHREN ZUR HERSTELLUNG VON MIRABEGRON-ENACARBIL
PROCÉDÉ DE PRÉPARATION DE MIRABEGRON ÉNACARBILE

(43) Date of publication of application: 14.10.2020
(73) Proprietor: Alfred E. Tiefenbacher (GmbH & Co. KG), 22767 Hamburg (DE)
(72) Inventor: Staver, Ruslan, 22767 Hamburg (DE); Sargsyan, Vardan, 0020 Yerevan (AM); Knyazyan, Aram, 0059 Yerevan (AM); Aghajanyan, Hasmik, 3902 Tavush Province (AM); Maier, Thomas, 78333 Stockach (DE)
(74) Representative: Hamm&Wittkopp Patentanwälte PartmbB

(56) References cited:
- EP-A1- 3 360 866
- "Chapter 11: TOOLS FOR PURIFYING THE PRODUCT: COLUMN CHROMATOGRAPHY, CRYSTALLIZATION AND RESLURRYING" In: ANDERSON: "PRACTICAL PROCESS RESEARCH AND DEVELOPMENT", 2000, ACADEMIC PRESS, SAN DIEGO, XP002382432, pages 223-247, * The whole document. In particular, pages 238-240, section III.C: Salt Selection. Cited as common general knowledge. *

## Description

The present invention relates to the prodrug mirabegron enacarbil and, in particular, to a process for its preparation.

Mirabegron is a β-3 adrenergic agonist indicated for the treatment of overactive bladder. Extended-release tablets containing 25 mg or 50 mg mirabegron are commercially available under the tradenames Betmiga^{®} and Myrbetriq^{®}. The use of mirabegron for the treatment of overactive bladder is described in European patent EP 1 559 427.

Initially, mirabegron was developed as a remedy for diabetes. European patent EP 1 028 111 discloses the preparation of the dihydrochloride salt of mirabegron (example 41) and its use for the treatment of diabetes mellitus. It is stated in European patent application EP 1 440 969 that the dihydrochloride salt has strong hygroscopicity and is unstable, which poses problems for converting the drug into solid unit dosage forms. As a solution to this problem, the application suggests the crystalline forms α and β of mirabegron (the free base), which are much less hygroscopic than the dihydrochloride salt.

The free base of mirabegron can be formulated as a stable matrix-type extended-release tablet containing polyethylene oxide as retarding polymer as well as ferric oxide, as described in European patent EP 1 205 190. However, the commercially available tablets, which are prepared by using the technique described in EP 1 205 190, provide a relatively low bioavailability of the drug, which is even lower if the tablet is taken together with food. Hence, there was a need for improving the oral bioavailability of mirabegron.

In order to enhance the oral bioavailability of mirabegron, European patent EP 3 360 866 suggests the use of a prodrug of mirabegron represented by the following formula 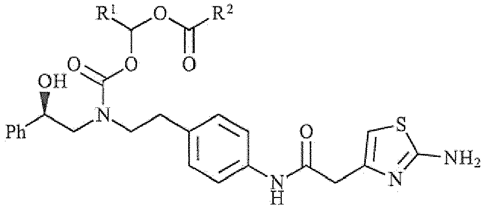 wherein R¹ represents hydrogen or alkyl, and R² represents alkyl, cycloalkyl, (cycloalkyl)alkyl, heterocyclyl, (heterocyclyl)alkyl, aryl or heteroaryl. Compared to the parent compound mirabegron, it was found that Tₘₐₓ is earlier and Cₘₐₓ is higher after oral administration of the prodrugs, in particular, after oral administration of the prodrug mirabegron enacarbil represented by the following formula I

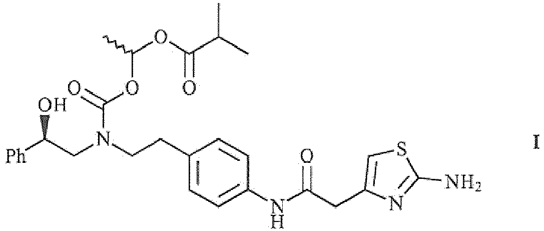

The manufacture of mirabegron enacarbil as described in example 1 of EP 3 360 866 is not suitable for an industrial scale production; preparative HPLC was used in order to purify the final product, which cannot easily be implemented on an industrial scale. Moreover, relatively expensive reagents were employed such as silver salts.

It was an object of the present invention to provide an improved process for the preparation of mirabegron enacarbil suitable for industrial scale production. This object is solved by the subject matter as defined in the claims.

In the process of the present invention, there is no need to purify mirabegron enacarbil by means of column chromatography or HPLC. Instead, the purification of mirabegron enacarbil is achieved by salt formation. The present invention relates to a process for preparing mirabegron enacarbil represented by the following formula I 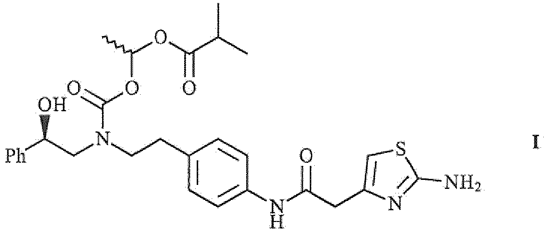 as a mixture of two diastereomers or as one diastereomer, wherein the process comprises the steps:
c) reacting mirabegron and 1-(isobutyryloxy)ethyl-p-nitrophenyl carbonate represented by the following formula (5) 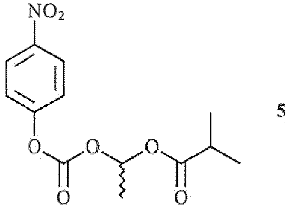 in a solvent, optionally in the presence of a base, followed by isolating crude compound I,
d) treating crude compound I in solution with phosphoric acid and isolating the precipitated phosphate salt of compound I, and
e) suspending the phosphate salt of compound I in water and a water-immiscible solvent, followed by extracting compound I into the water-immiscible solvent and removing the water-immiscible solvent to obtain compound I.

Preferably, compound 5 is prepared by reacting 1 -chloroethyl-p-nitrophenyl carbonate and isobutyric acid of the following formulas 3 and 4 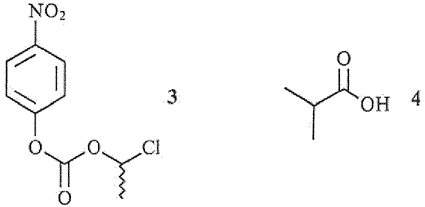 in the presence of a coupling agent.

Compound 3 may be prepared by reacting p-nitrophenol and 1-chloroethyl chloroformate of the following formulas 1 and 2 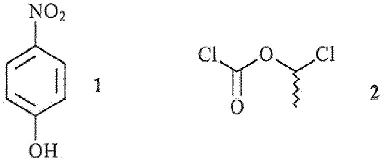 in a solvent, optionally in the presence of a base.

The process of the present invention is summarized in below reaction scheme.

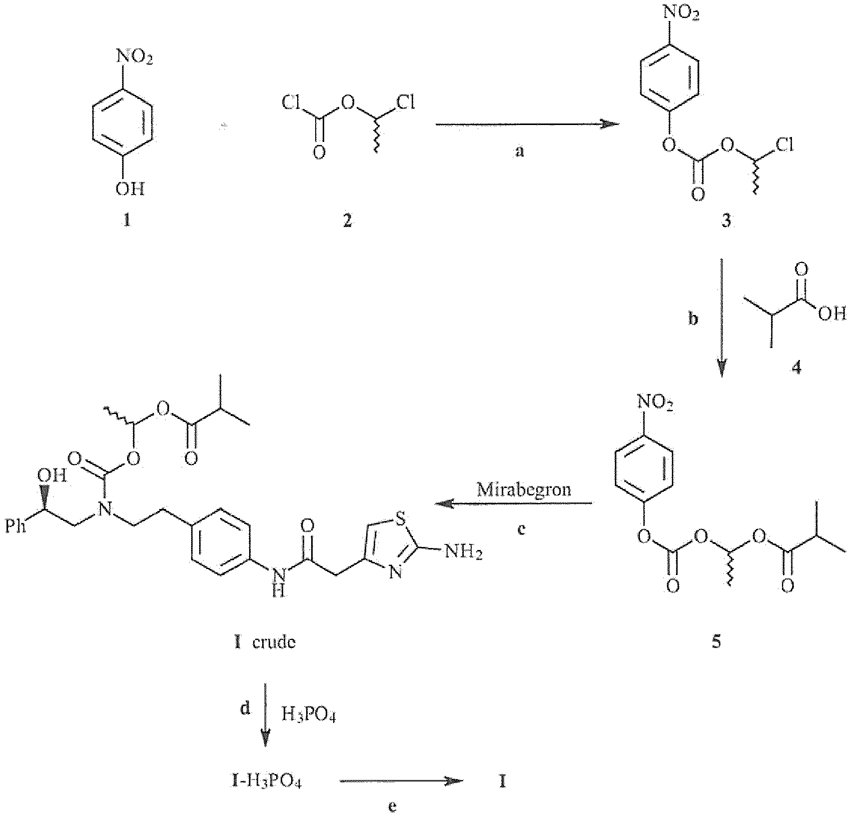

In method step a, bis(p-nitrophenyl)carbonate may be generated as by-product, the generation of which can be suppressed by preparing a solution of compounds 1 and 2 at a temperature of 15-25 °C, preferably 18-23 °C, followed by cooling the solution to -15 to 0 °C, preferably -15 to -10 °C, and adding a base, such as an inorganic base (e.g. sodium or potassium carbonate), preferably an amine base. Examples of suitable solvents include dichloromethane and tetrahydrofuran (THF), while examples of suitable amine bases include triethylamine, pyridine, dimethyl aminopyridine, aniline and 2,6-lutidine. Preferably, the reaction is conducted in THF with triethylamine, The coupling reaction proceeds rapidly at a temperature of 0 °C and below and is usually completed within 10 minutes after the addition of the base. Thus, in method step a, the addition of the base and the coupling reaction is preferably conducted at a temperature of -15 to 0 °C, more preferred -15 to -10 °C, in order to suppress the formation of the by-product bis(p-nitrophenyl)carbonate. Typically, the reaction mixture is filtered at a temperature of 0 °C and below within 10-30 min after the addition of the base, and crude compound 3 is isolated from the filtrate by evaporation of the solvent. Crude compound 3 may be purified by preparing a solution in a solvent such as THF and adding a hydrocarbon anti-solvent, preferably a saturated C₅₋₇ hydrocarbon such as n-heptane.

The reaction between compounds 3 and 4 is disclosed in US 8,283,487. The problem associated with the generation of compound 5 is the elimination of hydrogen chloride from compound 3 if an alkali metal or amine salt of compound 4 is generated in the reaction, e.g. if compound 4 is treated with sodium hydroxide or triethylamine. EP 3 360 866 suggests the use of silver carbonate as coupling agent, while according to US 8,283,487, Zn, Cu, Ce and Ni oxides and carboxylic acid salts are suitable as coupling agent. However, US 8,283,487 discloses that, if an oxide or a carboxylic acid salt such as the acetate (OAc) of Zn, Cu, Ce or Ni is used, it is preferred that a bromide or iodide catalyst such as NaI, NaBr, tetrabutylammonium bromide, tetrabutylammonium iodide, KBr, KI, LiBr or LiI is also present.

In the process of the present invention, method step b is preferably performed in the presence of a coupling agent selected from Cu(OAc)₂, Zn(OAc)₂, Cu₂O, CuO, CeO₂, NiO and ZnO, wherein ZnO is preferred.

It was found that no bromide or iodide catalyst is necessary in this reaction, so that method step b may be conducted in the presence of a Cu, Zn, Ce or Ni oxide or carboxylic acid salt coupling agent only, most preferably only in the presence of ZnO. On the other hand, the presence of a bromide or iodide catalyst slows down decomposition of compounds 3 and 5.

Method step b can be conducted in solution using, e.g. an aromatic solvent such as toluene. Preferably, the reaction is then conducted in the heat under reflux. However, it is preferred that no solvent is present, i.e. that the coupling reaction is performed in isobutyric acid, wherein the reaction is usually conducted at 50-90 °C, preferably 60-80 °C, and usually for 3-6 h, preferably 4-5 h. Longer reaction times result in the generation of compound 1 and p-nitrophenyl isobutyrate as by-products. Longer reaction times are only feasible in the presence of the bromide or iodide catalyst (8-12 h). If a Cu, Zn, Ce or Ni oxide is used as a coupling agent, a suspension is initially formed. It was found that the coupling reaction has been completed at this point in time when the reaction mixture became a solution indicating that the coupling agent was completely dissolved.

Method step c is performed in solution; suitable solvents are dichloromethane and THF. The reaction may be performed in the presence of a base such as triethylamine, pyridine, dimethyl aminopyridine, aniline, potassium carbonate or 2,6-lutidine. However, more preferred, the reaction is conducted in THF and in the absence of a base in order to prevent the formation of a 2-oxazolidinone by-product via an intramolecular cyclization between the prodrug's alcohol and carbamate groups.

Method step c is preferably conducted with a molar ratio of mirabegron to compound 5 of 1 : 1.10 to 1.10 : 1, more preferred 1 : 1.

In method step c, mirabegron enacarbil is obtained as crude product that contains, besides the starting material compound 1 and the intermediate compound 3, the by-products shown in below scheme:

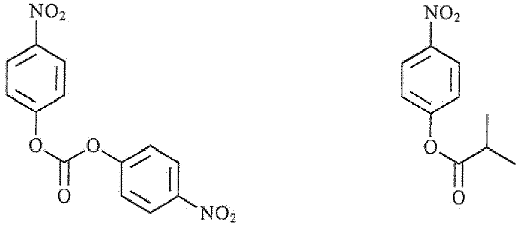

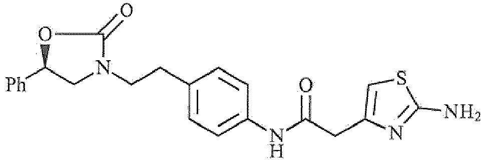

The formation of the 2-oxazolidinone by-product may be suppressed by adjusting the reaction conditions. However, the p-nitrophenol derivatives bis(p-nitrophenyl) carbonate and p-nitrophenyl isobutyrate as well as compounds 1 and 3 cannot be removed easily from compound I (crude). In example 1 of EP 3 360 866, preparative HPLC was used, whereas in the method disclosed in US 8,283,487, gabapentin enacarbil was purified by subjecting crude gabapentin enacarbil to catalytic hydrogenation (Pd/C) in order to reduce the nitro group in the by-products to an amino group, thereby generating aniline by-products, which can be separated from gabapentin enacarbil via salt formation and separating the salts by filtration or via acidic extraction. The latter purification method is not suitable for mirabegron enacarbil, because this prodrug contains an amino group that may form a salt as well.

Purification of mirabegron enacarbil by re-crystallization failed because this prodrug did not crystallize in various solvent systems; mirabegron enacarbil precipitates together with the by-products as an amorphous material. It is believed that the direct crystallization of mirabegron enacarbil for purification failed because the prodrug is obtained as a mixture of two diastereomers typically in a ratio of about 4 : 1 (as determined by HPLC and ¹H NMR). The chiral center of the enacarbil moiety is indicated in the above schemes by a zigzag bond, so that two diastereomers of mirabegron enacarbil are generated in the process of the present invention: *R*-hydroxy/*R*-methyl and *R*-hydroxy/*S*-methyl. It was therefore surprising that mirabegron enacarbil forms a phosphate salt in the reaction with phosphoric acid.

In method step d, crude compound I is dissolved in a solvent, e.g. methyl ethyl ketone or acetonitrile, followed by adding phosphoric acid, preferably a solution of 85 wt.-%. of phosphoric acid in water, at ambient temperature (18-25 °C). Alternatively, crude compound I is dissolved in a solvent, e.g. methyl ethyl ketone or acetonitrile which contain up to 5 % (w/v) phosphoric acid, at ambient temperature (18-25 °C). The precipitated phosphate salt of compound I (I-H₃PO₄) can be separated off by filtration.

In method step e, I-H₃PO₄ is suspended in water from which pure mirabegron enacarbil can be extracted into a water-immiscible solvent, which is preferably a carboxylic acid ester. Examples of suitable carboxylic acid esters include ethyl acetate, isopropyl acetate and n-butyl acetate, wherein ethyl acetate is preferred. The extraction may be facilitated by neutralizing the aqueous suspension of the phosphate salt with a base, e.g. with a saturated aqueous solution of sodium bicarbonate.

If desired, the diastereomers of mirabegron enacarbil may be separated by using preparative HPLC as disclosed in example 1 of EP 3 360 866.

The present invention also relates to a phosphate salt of mirabegron enacarbil, and to its use for purifying mirabegron enacarbil. In this process, mirabegron enacarbil is isolated as a phosphate salt by precipitation from a solution, the phosphate salt is optionally subjected to re-crystallization, and mirabegron enacarbil can be obtained by neutralizing an aqueous suspension of the phosphate salt with a base, e.g. with a saturated aqueous solution of sodium bicarbonate (until the pH is about 6), and extracting mirabegron enacarbil with a water-immiscible solvent.

Examples of solvents that may be used for re-crystallization of the phosphate salt of mirabegron enacarbil include acetonitrile, acetonitrile : water (e.g. 10 : 1, 15 : 1, 20 : 1, 25 : 1 (v/v)), acetone and methyl ethyl ketone.

The process of the present invention yields mirabegron enacarbil in a purity of at least 96 %, preferably at least 98 %, and more preferred at least 99 %, as determined by HPLC.

The invention is further illustrated by reference to the following examples.

### Examples

HPLC (for determining the purity of mirabegron enacarbil or the phosphate salt): Nucleodur^{©} PolarTec 250 × 4 mm, particle size 5 µm, carbon content 17%; 0.05% trifluoroacetic acid (TFA) in H₂O (eluent A), 0.05% TFA in acetonitrile (eluent B); gradient 5-95% B; flow rate 0.7 ml/min.

### Example 1. 1-Chloroethyl-p-nitrophenyl carbonate (compound 3)

30 g p-Nitrophenol (1 eq.) have been suspended in 650 mL THF. Into the obtained mixture 26.38 mL (1.13 eq) 1-chloroethyl chloroformate have been added at room temperature. After cooling to -10 °C, 59.6 mL triethylamine (2 eq.) have been added dropwise to keep temperature below -10 °C. Reaction mixture was stirred at -10 °C for 2 h. After 2 h, reaction mixture was heated to room temperature and 300 mL dichloromethane have been added. After filtration of reaction mixture, the filtrate was washed with saturated NaHCO₃ solution until full removal of residual p-nitrophenol. (colour test / up to 15 l NaHCO₃ were used). The organic layer was dried over MgSO₄, and evaporated under vacuum to yield 45.85 g crude product (86.8 % yield) in 94 % purity (HPLC) as pale yellow solid.

### Example 2. 1-Chloroethyl-p-nitrophenyl carbonate (compound 3)

p-Nitrophenol (200 g, 1 eq.) was dissolved in THF (4 L) in a N₂ atmosphere and the transparent solution was cooled to -12°C. To the resulting solution 1-chloroethyl chloroformate (175 ml, 1.13 eq) was added and followed by addition of triethyl amine (303 ml, 1.5 eq.) in portions. After completion of the addition, the internal temperature of the obtained green yellowish suspension increased to 18°C. The resulting suspension was cooled to -12°C again and stirred for 20 min at -12°C. The suspension was filtered at this temperature, and the filter cake was washed with THF (250 ml). The filtrates were combined, and the solvent was removed by using a rotary evaporator at 40°C. Compound 3 (336 g, yield 95 %) was obtained as a milky white solid with a purity of 96 % (HPLC).

### Example 3. 1-(Isobutyryloxy)ethyl-p-nitrophenyl carbonate (compound 5)

1 g Compound 3 (1 eq.), 0.33 g ZnO (1 eq.) and 0.33 g KI (0.5 eq.) have been suspended in 8 mL (21.6 eq.) isobutyric acid and heated to 80 °C. Reaction mixture was stirred for 10-12 h until detected content of starting material was below 10 %. Reaction mixture was cooled to room temperature, and 40 mL dichloromethane have been added. Obtained solution was washed with 50 mL water and 500 mL saturated NaHCO₃ solution until full removal of nitrophenol by-product (colour test). The organic layer was dried over MgSO₄, and evaporated under vacuum to yield 0.58 g crude product (48 % yield) in 82 % purity (HPLC) as a pale yellow to orange solid.

### Example 4. 1-(Isobutyryloxy)ethyl-p-nitrophenyl carbonate (compound 5)

Compound 3 (350 g, 1 eq.), ZnO (1eq) were suspended in isobutyric acid (15 eq.) at ambient temperature in a N₂ atmosphere and heated to 80°C. The obtained suspension was stirred for 4-5 h at 80°C until ZnO was completely dissolved. The obtained dark brown solution was cooled to room temperature and was washed with a large excess of a saturated aqueous solution of sodium bicarbonate and solid bicarbonate powder until the gas release (CO₂) stopped, because excess isobutyric acid was neutralized (120 L saturated solution and about 300 g solid bicarbonate were used). The residual organic layer was dried with MgSO₄, the solvent was removed by using a rotary evaporator. Crude compound 5 was obtained as a dark brown, viscous oil (274 g; 64 % yield) in 59 % purity (HPLC).

### Example 5. Mirabegron enacarbil (crude)

9.9 g Mirabegron (1 eq.) and 10 g compound 5 (1 eq., HPLC purity = 72%) have been dissolved in 150 mL THF. Into obtained solution, 9,5 mL 2,6-lutidine (5 eq.) have been added and reaction mixture was heated to 40 °C. Stirring of reaction mixture continued for 6 h until content of starting materials < 5 %. Then, the reaction mixture was cooled to room temperature, and 300 mL dichloromethane have been added. Obtained solution was washed with saturated NaHCO₃ solution until full removal of the nitrophenol by-products (colour test / up to 8000 mL NaHCO₃ solution was used). The organic layer was dried over MgSO₄, and evaporated under vacuum to yield 13.66 g crude prodrug I (117 % yield) as a dark brown, viscous oil in 66% purity (HPLC).

### Example 6. Mirabegron enacarbil (crude)

Compound 5 (170 g, 1 eq.) and mirabegron (1 eq.) were suspended in THF (3500 ml) and heated for 3-4 h. The obtained dark brown solution was cooled to ambient temperature, and the white precipitate (unreacted mirabegron) was removed by filtration. The filtrate was diluted with ethyl acetate (2000 ml), washed with 1 M HCl solution (2000 ml) and then neutralized with a saturated aqueous solution of sodium bicarbonate (3000 ml). The organic layer was dried with MgSO₄, and the solvent was removed by using a rotary evaporator to yield 150 g crude compound I (78 %) as a dark brown viscous oil in a purity of 75 % (HPLC).

### Example 7. Mirabegron enacarbil phosphate

Crude prodrug I was dissolved in 200 mL 0.85 % orthophosphoric acid solution in acetonitrile (200 mL solution of 2% w/v phosphoric acid in acetonitrile was prepared by dissolving 4.7 g 85 wt. % aqueous phosphoric acid solution in 196 mL acetonitrile). After 30 min stirring, precipitation started and stirring was continued for additional 2 h. Obtained precipitate was filtered off and 8.47 g phosphate salt of mirabegron enacarbil phosphate salt was isolated in 72% overall yield and 96-98 % purity (HPLC).

### Example 8. Mirabegron enacarbil

The phosphate salt of prodrug I (140 g) was suspended in H₂O : EtOAc (1 : 2) and neutralized with a saturated aqueous solution of sodium bicarbonate (700 ml) until the pH was adjusted to 6. The obtained transparent organic layer was separated and dried with MgSO4. The solvent was removed by using a rotary evaporator to yield mirabegron enacarbil (106 g) as a pale yellow solid with a purity of 95 % (HPLC).

## Claims

1. A process for preparing mirabegron enacarbil represented by the following formula I 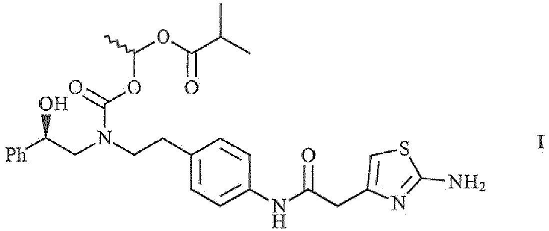 as a mixture of two diastereomers or as one diastereomer, wherein the process comprises the steps:
c) reacting mirabegron and 1-(isobutyryloxy)ethyl-p-nitrophenyl carbonate represented by the following formula (5) 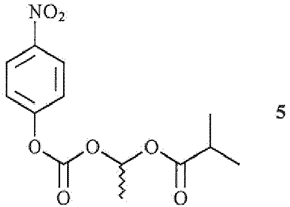 in a solvent, optionally in the presence of a base, followed by isolating crude compound I,
d) treating crude compound I in solution with phosphoric acid and isolating the precipitated phosphate salt of compound I, and
e) suspending the phosphate salt of compound I in water and a water-immiscible solvent, followed by extracting compound I into the water-immiscible solvent and removing the water-immiscible solvent to obtain compound I.

2. The process according to claim 1, wherein compound 5 is prepared by reacting 1-chloroethyl-p-nitrophenyl carbonate and isobutyric acid of the following formulas 3 and 4 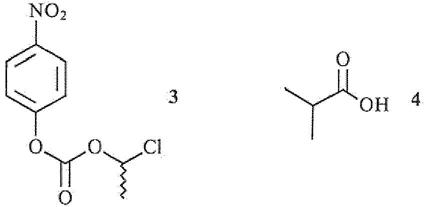 in the presence of a coupling agent.

3. The process according to claim 2, wherein the coupling agent is selected from Cu(OAc)₂, Zn(OAc)₂, Cu₂O, CuO, CeO₂, NiO and ZnO, wherein ZnO is preferred.

4. The process according to claim 2 or 3, wherein compound 3 is prepared by reacting p-nitrophenol and 1-chloroethyl chloroformate of the following formulas 1 and 2 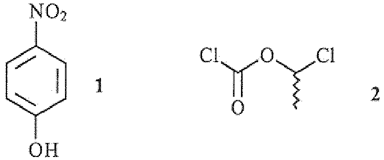 in a solvent, optionally in the presence of a base.

5. The process according to any one of the preceding claims, wherein the molar ratio of mirabegron to compound 5 in step c is 1 : 1.10 to 1.10 : 1, preferably 1 : 1.

6. The process according to any one of the preceding claims, wherein step c is conducted in the absence of a base.

7. The process according to any one of the preceding claims, wherein the solvent in step c is tetrahydrofuran.

8. The process according to any one of the preceding claims, wherein the solvent in step d is acetonitrile.

9. The process according to any one of the preceding claims, wherein the water-immiscible solvent in step e is a carboxylic acid ester.

10. The process according to claim 9, wherein the carboxylic acid ester is selected from ethyl acetate, isopropyl acetate and n-butyl acetate, wherein ethyl acetate is preferred.

11. A phosphate salt of mirabegron enacarbil.

12. A process for purifying mirabegron enacarbil, in which mirabegron enacarbil is isolated as a phosphate salt by precipitation from a solution, the phosphate salt is optionally subjected to recrystallization, and mirabegron enacarbil is extracted from an aqueous suspension of the phosphate salt with a water-immiscible solvent.

13. The process according to claim 12, wherein the mirabegron enacarbil isolated from the extract has a purity of at least 96 %, preferably at least 98 %, and more preferred at least 99 %, as determined by HPLC.

## Patentansprüche

1. Verfahren zur Herstellung von Mirabegronenacarbil der nachstehenden Formel I 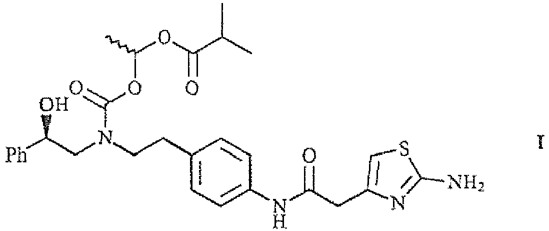 als ein Gemisch zweier Diastereomere oder als ein Diastereomer, wobei das Verfahren die Schritte umfasst:
c) Umsetzen von Mirabegron mit 1-(Isobutyryloxy)ethyl-p-nitrophenylcarbonat der nachstehenden Formel (5) 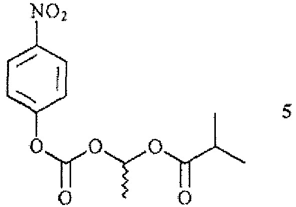 in einem Lösungsmittel, gegebenenfalls in Gegenwart einer Base, gefolgt von Isolieren der Verbindung I als Rohprodukt,
d) Behandeln des Rohprodukts der Verbindung I in einer Lösung mit Phosphorsäure und Isolieren des ausgefällten Phosphatsalzes der Verbindung I,
e) Suspendieren des Phosphatsalzes der Verbindung I in Wasser und einem mit Wasser nicht mischbaren Lösungsmittel, gefolgt von Extrahieren der Verbindung I in das mit Wasser nicht mischbare Lösungsmittel und Entfernen des mit Wasser nicht mischbaren Lösungsmittels, um die Verbindung I zu erhalten.

2. Verfahren gemäß Anspruch 1, wobei Verbindung 5 hergestellt wird durch Umsetzen von 1-Chlorethyl-p-nitrophenylcarbonat mit Isobuttersäure der nachstehenden Formeln 3 und 4 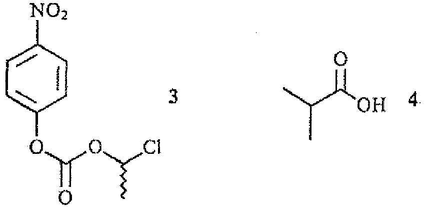 in Gegenwart eines Kupplungsreagenzes.

3. Verfahren gemäß Anspruch 2, wobei das Kupplungsreagenz ausgewählt ist aus Cu(OAc)₂, Zn(OAc)₂, Cu₂O, CuO, CeO₂, NiO und ZnO, wobei ZnO bevorzugt ist.

4. Verfahren gemäß Anspruch 2 oder 3, wobei Verbindung 3 hergestellt wird durch Umsetzen von p-Nitrophenol mit 1-Chlorethylchlorformiat der nachstehenden Formeln 1 und 2 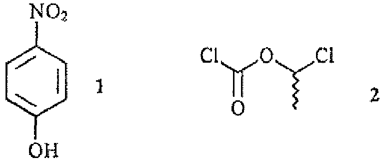 in einem Lösungsmittel, gegebenenfalls in Gegenwart einer Base.

5. Verfahren gemäß einem der vorstehenden Ansprüche, wobei das molare Verhältnis von Mirabegron zur Verbindung 5 im Verfahrensschritt c 1:1.10 bis 1.10:1 ist, vorzugsweise 1:1.

6. Verfahren gemäß einem der vorstehenden Ansprüche, wobei Verfahrensschritt c in Abwesenheit einer Base durchgeführt wird.

7. Verfahren gemäß einem der vorstehenden Ansprüche, wobei das Lösungsmittel im Verfahrensschritt c Tetrahydrofuran ist.

8. Verfahren gemäß einem der vorstehenden Ansprüche, wobei das Lösungsmittel im Verfahrensschritt d Acetonitril ist.

9. Verfahren gemäß einem der vorstehenden Ansprüche, wobei das mit Wasser nicht mischbare Lösungsmittel im Verfahrensschritt e ein Carbonsäureester ist.

10. Verfahren gemäß Anspruch 9, wobei der Carbonsäureester ausgewählt ist aus Ethylacetat, Isopropylacetat und n-Butylacetat, wobei Ethylacetat bevorzugt ist.

11. Phosphatsalz von Mirabegronenacarbil.

12. Verfahren zur Reinigung von Mirabegronenacarbil, in dem Mirabegronenacarbil als Phosphatsalz durch Fällung aus einer Lösung isoliert wird, das Phosphatsalz gegebenenfalls umkristallisiert und Mirabegronenacarbil aus einer wässrigen Suspension des Phosphatsalzes mit einem mit Wasser nicht mischbaren Lösungsmittel extrahiert wird.

13. Verfahren gemäß Anspruch 12, wobei das aus dem Extrakt isolierte Mirabegronenacarbil eine Reinheit von mindestens 96%, vorzugsweise mindestens 98% und mehr bevorzugt mindestens 99% aufweist, bestimmt mittels HPLC.

## Revendications

1. Procédé de préparation de mirabégron énacarbil représenté par la formule I suivante 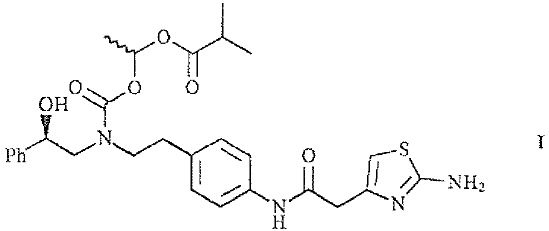 sous forme d'un mélange de deux diastéréomères ou sous forme d'un diastéréomère, dans lequel le procédé comprend les étapes consistant à
c) faire réagir du mirabégron et du carbonate de 1-(isobutyryloxy)éthyl-p-nitrophényle représenté par la formule (5) suivante 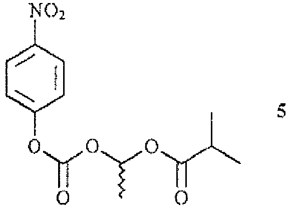 dans un solvant, facultativement en présence d'une base, puis isoler le composé brut I,
d) traiter le composé brut I en solution avec de l'acide phosphorique et isoler le sel de phosphate précipité du composé I, et
e) mettre en suspension le sel de phosphate du composé I dans de l'eau et un solvant non miscible à l'eau, puis extraire le composé I dans le solvant non miscible à l'eau et éliminer le solvant non miscible à l'eau pour obtenir le composé I.

2. Procédé selon la revendication 1, dans lequel le composé 5 est préparé en faisant réagir du carbonate de 1-chloroéthyl-p-nitrophényle et de l'acide isobutyrique des formules 3 et 4 suivantes 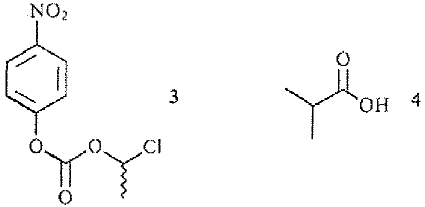 en présence d'un agent de couplage.

3. Procédé selon la revendication 2, dans lequel l'agent de couplage est choisi parmi Cu(OAc)₂, Zn(OAc)₂, Cu₂O, CuO, CeO₂, NiO et ZnO, dans lequel ZnO est préféré.

4. Procédé selon la revendication 2 ou 3, dans lequel le composé 3 est préparé en faisant réagir du p-nitrophénol et du chloroformate de 1-chloroéthyle des formules 1 et 2 suivantes 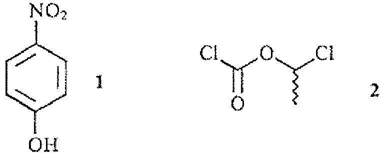 dans un solvant, facultativement en présence d'une base.

5. Procédé selon l'une quelconque des revendications précédentes, dans lequel le rapport molaire entre le mirabégron et le composé 5 à l'étape c est de 1:1,10 à 1,10:1, de préférence 1:1.

6. Procédé selon l'une quelconque des revendications précédentes, dans lequel l'étape c est effectuée en l'absence d'une base.

7. Procédé selon l'une quelconque des revendications précédentes, dans lequel le solvant de l'étape c est du tétrahydrofuranne.

8. Procédé selon l'une quelconque des revendications précédentes, dans lequel le solvant de l'étape d est de l'acétonitrile.

9. Procédé selon l'une quelconque des revendications précédentes, dans lequel le solvant non miscible à l'eau à l'étape e est un ester d'acide carboxylique.

10. Procédé selon la revendication 9, dans lequel l'ester d'acide carboxylique est choisi parmi de l'acétate d'éthyle, acétate d'isopropyle et acétate de n-butyle, dans lequel de l'acétate d'éthyle est préféré.

11. Sel phosphaté de mirabégron énacarbil.

12. Procédé de purification de mirabégron énacarbil, dans lequel le mirabégron énacarbil est isolé sous la forme d'un sel phosphaté par précipitation à partir d'une solution, le sel phosphaté est facultativement soumis à une recristallisation, et le mirabégron énacarbil est extrait d'une suspension aqueuse du sel phosphaté avec un solvant non miscible à l'eau.

13. Procédé selon la revendication 12, dans lequel le mirabégron énacarbil isolé à partir de l'extrait a une pureté d'au moins 96 %, de préférence d'au moins 98 %, et de manière plus préférée d'au moins 99 %, comme déterminé par HPLC.
